# EUROPEAN PATENT APPLICATION

(11) **EP 1 380 294 A1**
(43) Date of publication of application: **14.01.2004**
(21) Application number: 02717106.5
(22) Date of filing: 11.04.2002
(51) Int. Cl.: A61K 31/255, A61K 7/16, A61K 9/08, A61K 9/12, A61K 9/20, A61K 9/70, A61P 1/02, A61P 29/00

(54) **PREVENTIVE/REMEDIAL AGENT FOR INFLAMMATORY DISEASE IN ORAL-CAVITY MUCOSA AND THE LIKE**

(30) Priority: 17.04.2001 JP 2001118113
(71) Applicant: TANABE SEIYAKU CO., LTD., Chuo-ku, Osaka-shi, Osaka 541-8505 (JP)
(72) Inventor: KIMOTO, Yasuhiko, Ikoma-shi, Nara 630-0121 (JP)
(74) Representative: HOFFMANN EITLE
(86) International application number: PCT/JP2002/003589
(87) International publication number: WO 2002/085347

(57) **Abstract**

A preventive/remedy for inflammatory diseases in mucosa of oral cavity, pharyngeal or laryngeal mucosa which contains as an active ingredient the sulfodehydroabietic acid represented by the formula (I): 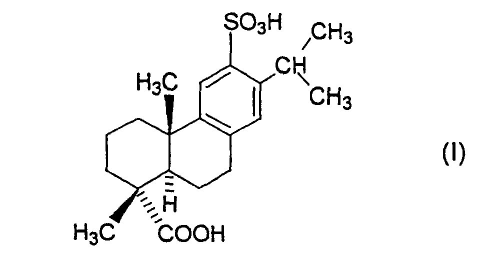 or a pharmaceutically acceptable salt thereof.

## Description

### TECHNICAL FIELD

The present invention relates to an agent for prophylaxis or'treatment of inflammatory diseases in mucosa of oral cavity, pharynx or larynx containing sulfodehydroabietic acid of the following formula (I): 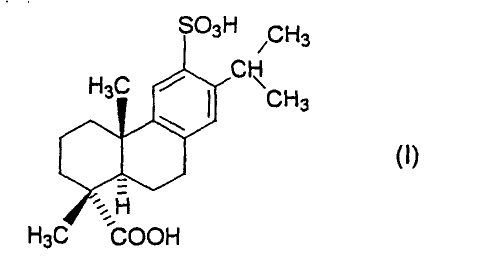 or a pharmaceutically acceptable salt thereof as an active ingredient.

### BACKGROUND ART

Inflammatory diseases in mucosa of oral cavity, pharynx or larynx are ones due to the response to intrinsic (internal) or extrinsic (external) stimulations including intrinsic causes such as autoimmune or influence from other regions. Oral cavity, pharynx and larynx are an entrance of digestive tract or respiratory organ and are susceptible to many stimulations and injuries and causes many kinds of inflammation.

Steroids, iodines, antibacterial agents and nonsteroidal anti-inflammatory agents have been used as an agent for prophylaxis or treatment of inflammatory diseases in mucosa of oral cavity, pharynx or larynx.

On the other hand, sulfodehydroabietic acid (I) or a salt thereof has been known to exhibit an inhibitory activity of acid secretion or pepsin secretion, etc., and to be useful as an agent for prophylaxis or treatment of peptic ulcer (gastric ulcer, duodenal ulcer) or gastritis (Japanese Patent Publication A 58-77814, Japanese Patent Publication A 63-165361 and Japanese Patent Publication A 2-167258).

### DISCLOSURE OF INVENTION

An object of the present invention is to provide a novel agent being useful in the prophylaxis or treatment of inflammatory diseases in mucosa of oral cavity, pharynx or larynx, especially to provide an agent showing its effect by directly contacting the agent with mucosa of oral cavity, pharynx or larynx.

During the studies on a novel remedy for inflammatory diseases in mucosa of oral cavity, pharynx or larynx, the present inventors have found that sulfodehydroabietic acid (I) or a pharmaceutically acceptable salt thereof exhibits an excellent effect in the prophylaxis or treatment of inflammatory diseases in mucosa of oral cavity, pharynx or larynx, and have accomplished the present invention.

### MODE FOR CARRYING OUT THE INVENTION

That is, the present invention relates to an agent for prophylaxis or treatment of inflammatory diseases in mucosa of oral cavity, pharynx or larynx, which comprises as an active ingredient sulfodehydroabietic acid (chemical name: (+)-(1R, 4aS, 10aR)-1,2,3,4,4a,9,10,10a-octahydro-1,4a-dimethyl-7-(1-methylethyl)-6-sulfo-1-phenanthrenecarboxylic acid) of the following formula (I): 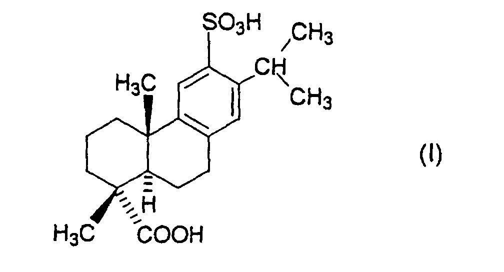 or a pharmaceutically acceptable salt thereof.

The present invention also relates to a use of said sulfodehydroabietic acid (I) or a pharmaceutically acceptable salt thereof in the preparation of an agent for prophylaxis or treatment of inflammatory diseases in mucosa of oral cavity, pharynx or larynx.

Moreover, the present invention relates to a method for prophylaxis or treatment of inflammatory diseases which comprises administering sulfodehydroabietic acid (I) or a pharmaceutically acceptable salt thereof to human beings especially in the form of the preparation directly contacting to mucosa of oral cavity, pharynx or larynx being suffering from an inflammatory disease, on said region.

The present invention relates, more concretely, to the agent for prophylaxis or treatment of inflammatory diseases wherein said diseases are aphtha and/or ulcer in mucosa of oral cavity, stomatitis, aphthous stomatitis and pharyngitis, a use in the preparation of the agent for said diseases, and a method for treating said diseases by using the agent, too.

Sulfodehydroabietic acid (I) or a pharmaceutically acceptable salt thereof used as an active ingredient of an agent for prophylaxis or treatment of inflammatory diseases in mucosa of oral cavity, pharynx or larynx is known and is prepared by methods described in Japanese Patent Publication A 58-77814, Japanese Patent Publication A 63-165361, Japanese Patent Publication A 2-167258 or by the similar method thereof.

The pharmaceutically acceptable salt of sulfodehydroabietic acid of the formula (I) includes, for example, a salt with an alkali metal (e.g., sodium, lithium, potassium, etc.), a salt with an alkaline earth metal (e.g., magnesium, calcium, etc.), and a salt with a metal such as aluminum. Among them, a preferable salt is sodium salt of sulfodehydroabietic acid, especially monosodium salt or disodium salt thereof, and the most preferable salt is sulfodehydroabietic acid monosodium salt.

Sulfodehydroabietic acid monosodium salt is more advantageous than disodium salt thereof as being less hygroscopic and being more stable (Japanese Patent Publication A 63-165361). Besides, a pharmaceutically acceptable salt of sulfodehydroabietic acid (I) may exist as well in the form of a hydrate thereof, and the hydrate of sulfodehydroabietic acid monosodium salt may be, for example, pentahydrate thereof, i.e., sulfodehydroabietic acid monosodium salt pentahydrate. The monosodium salt pentahydrate of sulfodehydroabietic acid of the formula (I) (chemical name: (+)-(1R,4aS,10aR)-1,2,3,4,4a,9,10,10a-octahydro-1,4a-dimethyl-7-(1-methylethyl)-6-sulfo-1-phenanthrene carboxylic acid 6-sodium salt pentahydrate) has been known as Ecabet sodium (generic name).

Sulfodehydroabietic acid (I) or a pharmaceutically acceptable salt thereof of the active ingredient of the present invention adheres to the inflammatory lesion of mucosa of oral cavity and so on, and exhibit the effect. Therefore, the agent is very excellent in the effect.

In addition, sulfodehydroabietic acid (I) or a pharmaceutically acceptable salt thereof of the active ingredient shows few side effects, and the safety thereof is extremely high.

It is especially desirable that sulfodehydroabietic acid (I) or a pharmaceutically acceptable salt thereof of the active ingredient of the present invention is administered in the form of the preparation so as to directly contact to the inflammatory lesion in mucosa of oral cavity, pharynx or larynx and for a long term.

There are illustrated such known preparations as gargles, liniments (e.g., ointments, creams, granules, fine granules, powders), patches (e.g., plasters, adhesive tapes), sprays, tablets (e.g., troches, buccals, degredative agents in oral cavity, sustained release preparation in oral cavity) and so on.

The present agent for prophylaxis or treatment of inflammatory in oral cavity, pharynx or larynx is preferably administered in such an administration route and a preparation as mentioned above.

The preparations mentioned above may contain a pharmaceutically acceptable carriers or excipients.

The gargles are either solutions or suspensions. Pharmaceutically acceptable carriers or excipients used in the gargles include, such as an aqueous medium (e.g., water), suspensions (e.g., acacia, gelatin, methylcellulose, carboxymethylcellulose sodium, hydroxymethylcellulose, aluminum stearate gel), surfactants (e.g., lecithin, sorbitan monooreate, glycerin monostearate), non-aqueous vehicles (e.g., glycerin, propylene glycol, vegetable oil) and so on. Furthermore, the gargles may contain preservations (e.g., methyl p-hydroxybenzoate, propyl p-hydroxybenzoate), perfuming agents and/or coloring agents. Granules, fine granules or powders prepared by the conventional method may be dissolved or suspended in water just before administration and used as the gargles.

The ointments can be prepared using known bases, such as plastibase, white vaseline, paraffin, polyethylene glycol(PEG), propylene glycol(PG), stearyl alcohol, stearic acid, bees wax, etc.

The tablets, such as troches, buccals, disintegrated agents in oral cavity, or sustained release preparations in oral cavity can be prepared by the conventional method using known excipients or carriers, such as binding agents (e.g., acacia, gelatin, dextrin, hydroxypropylcellulose, methylcellulose, polyvinylpyrrolidone), diluents (e.g., lactose, sucrose, mannitol, corn starch, potato starch, calcium phosphate, calcium citrate, crystalline cellulose), lubricants (e.g., magnesium stearate, calcium stearate, stearic acid, talc, anhydrous silicic acid), disintegrants (e.g., corn starch, potato starch, carboxymethylcellulose, carboxymethylcellulose calcium, alginic acid), wetting agents (e.g., sodium laurylsulfate) and the like.

The other preparations can be also prepared by the known conventional method using known exipients, etc.

The dose of the compound (I) or a pharmaceutically acceptable salt thereof of the active ingredient of the present agent may vary according to the administration route, age of a patient, or severity of the disease to be cured, but the daily dose thereof for an adult is usually in the range of about 10 mg to 300 mg/kg, preferably in the range of about 20 mg to 300 mg/kg, especially in the range of 50 mg/kg to 200 mg/kg.

In the present specification, the term "prophylaxis or treatment" comprises the improvement of symptoms such as pain, the prevention of exacerbation, the maintenance of remission, the prevention of recrudescence.

### EXAMPLES

The present agent and its efficacy will be illustrated in more detail by the following Experiments and Preparations.

### Experiment 1

A woman (30 years old) was suffering from aphthous stomatitis for 5 to 6 days:

Granules containing 66.7% of Ecabet sodium (Trade name Gastrome: Tanabe Seiyaku) were directly applied to the lesion of the patient. After 5 hours of the application, pains were disappeared and the membrane was formed on the lesion. On the next day aphtha was healed:

### Experiment 2

A man (72 years old) had ulcer after biting labium and the ulcer did not disappear for 1 week:

Granules containing 66.7% of Ecabet sodium (Trade name Gastrome: Tanabe Seiyaku) was directly applied to the lesion of the patient noon on that day. The pains were disappeared at night on that day and on the next day the ulcer was healed:

### Preparation 1

Ecabet sodium (700 g), D-mannitol (252.7 g), sodium chloride (20 g), aspartame (5 g) and magnesium stearate (20 g) were granulated by a wet granulator, and thereto were added L-menthol (0.3 g) and hydrous silicon dioxide (2 g), and the mixture was mixed to give granules.

### Preparation 2

To Ecabet sodium (700 g), D-mannitol (255 g), sodium chloride (20 g), aspartame (5 g) and magnesium stearate (20 g) were added water, and the mixture was granulated by a wet granulator to give granules.

### Preparation 3

Ecabet sodium (700 g), D-mannitol (175 g), sodium chloride (105 g) and magnesium stearate (20 g) were mixed to give powders.

### Preparation 4

Ecabet sodium (700 g), D-mannitol (265.8 g), sodium chloride (7 g), aspartame (5 g) and magnesium stearate (20 g) were granulated by a wet granulator, and thereto were added L-menthol (0.3 g) and hydrous silicon dioxide (2 g), and the mixture was mixed, and compressed with a tableting machine to give tablets.

### Preparation 5

Ecabet sodium (700 g), D-mannitol (242.7 g), potassium chloride (30 g), aspartame (5 g) and magnesium stearate (20 g) were granulated with a wet granulator, and thereto were added 1-menthol (0.3 g) and hydrous silicon dioxide (2g). The mixture was mixed to give granules.

### Preparation 6

The preparation (1.5g) obtained in Preparation 1 was suspended in water (100ml) to give gargles.

### Preparations 7 to 9

The macrogol ointments respectively, having ingredients shown in Table 1 were prepared as following procedure.
(1) PEG 400 (Nippon Soda Co., Ltd.)and PEG 4000 (Sanyo Chemical Ind.) were prepared in the ratio of 6 to 4 and each was heated about 70°C.
(2) Next, Ecabet sodium in the amount of shown in Table 1 was added to PEG 400 prepared in (1).
(3) Then, PEG 4000 was added thereto in the amount shown in Table 1 and the mixture was stirred by an ultramixer while warming at 70C°.
(4) Further the mixture was continued to stir and was gradually cooled to room temperature to give macrogol ointments.

**Table 1**

| Preparation number | | 7 | 8 | 9 |
|---|---|---|---|---|
| Ingredient(g) | Ecabet sodium | 2 | 6 | 10 |
| | PEG 400 | 58.8 | 56.4 | 54 |
| | PEG 4000 | 39.2 | 37.6 | 36 |

### Preparations 10 to 11

The creams respectively, having ingredients shown in Table 2 were prepared as following procedure.
(1) Propylene glycol was previously heated at 70 - 80°C. Thereto was added Ecabet sodium and the mixture was well kneaded.
(2) Next, to the mixture prepared in (1) were added methyl p-hyoxybenzoate, propyl p-hydroxybenzoate and water which were previously warmed at 75°C.
(3) To the mixture prepared in (2) were added stearyl alcohol, white vaseline, polyoxyetylene hydrogenated castor oil and glycerylmonostearate which were previously warmed at 75°C and the mixture was stirred by an ultramixer while warming at 75°C.
(4) The mixture was continued to stir and was gradually cooled to room temperature to give creams.

**Table 2**

| Preparation Number | | 10 | 11 |
|---|---|---|---|
| Ingredient (g) | Ecabet sodium | 2 | 6 |
| | propylene glycol | 12 | 12 |
| | Stearyl alcohol | 20 | 20 |
| | White vaseline | 25 | 25 |
| | Polyoxyethylene (60) hydrogenated castor oil | 4 | 4 |
| | Glyceryl monostearate | 1 | 1 |
| | Methyl p-hydroxybenzoate | 0.1 | 0.1 |
| | Propyl p-hydroxybenzoate | 0.1 | 0.1 |
| | Water | 35.1 | 31.8 |

### Preparations 12 to 13

FAPG bases respectively, having ingredients shown in Table 3 were prepared as following procedure.
(1) Stearyl alcohol and stearic acid were previously heated at 80 - 85°C.
(2) Next, Ecabet sodium was added to PG and the mixture was warmed at 75°C to dissolve.
(3) Then, the warmed mixture prepared in (1) and the mixture prepared in (2) were mixed, and the mixture was stirred by an ultramixer while warming at 75°C.
(4) The mixture was continued to stir and was rapidly cooled to room temperature to give FAPG bases.

**Table 3**

| Preparation Number | | 12 | 13 |
|---|---|---|---|
| Ingredient (g) | Ecabet sodium | 2 | 6 |
| | Stearyl alcohol | 25 | 25 |
| | Stearyic acid | 5 | 5 |
| | Propylene glycol | 68 | 64 |

### Preparations 14 to 15

Vaseline ointment respectively, having ingredients shown in Table 4 were prepared as following procedure.
(1) Stearyl alcohol and bees wax were mixed and previously warmed at about 70 - 80°C.
(2) Then, Ecabet sodium was added to the mixture prepared in (1) and the mixture was melted at 75°C and kept at about 70 - 80°C.
(3) White vaseline which was previously warmed at about 70-80C was added to the mixture prepared in (2) and the mixture was stirred by an ultra mixer while warming at 75°C.
(4) The mixture was continued to stir and was cooled to room temperature to give vaseline ointments.

**Table 4**

| Preparation Number | | 14 | 15 |
|---|---|---|---|
| Ingredient (g) | Ecabet sodium | 2 | 6 |
| | Bees wax | 8 | 8 |
| | Srearyl alcohol | 3 | 3 |
| | White vaseline | 87 | 83 |

As is clear from the above result, vaseline ointments can be easily prepared.

### Preparations 16 to 17

The emollient base creams respectively, having ingredients shown in Table 5 were prepared as following procedure.
(1) Propylene glycol, stearyl alcohol, cetanol, liquid paraffin, polyoxyl stearate, sorbitan fatty acid ester and purified water were previously warmed at 70 - 80°C, respectively.
(2) Next, Ecabet sodium was added to propylene glycol and the mixture was warmed at 70 - 80°C to melt.
(3) Then, each ingredient except for propylene glycol and the mixture prepared in (2) were mixed, and the mixture was stirred by ultramixer while warming at 75°C.
(4) The mixture was continued to stir and was gradually cooled to room temperature to give emollient base creams.

**Table 5**

| Preparation Number | | 16 | 17 |
|---|---|---|---|
| Ingredient (g) | Ecabet sodium | 2 | 6 |
| | Stearyl alcohol | 6 | 6 |
| | Cetanol | 4 | 4 |
| | propylene glycol | 30 | 30 |
| | liquid paraffin | 3 | 3 |
| | Polyoxyl stearate 40 | 5 | 5 |
| | Sorbitan sesquioleate | 2 | 2 |
| | Purified water | 48 | 44 |

### INDUSTRIAL APPLICABILITY

Sulfodehydroabietic acid or a pharmaceutically acceptable salt thereof of the active ingredient of the present invention is useful in the prophylaxis or treatment of inflammatory diseases in mucosa of oral cavity, pharynx or larynx.

## Claims

1. An agent for prophylaxis or treatment of inflammatory diseases in mucosa of oral cavity, pharynx or larynx, which comprises as an active ingredient sulfodehydroabietic acid represented by the formula (I): 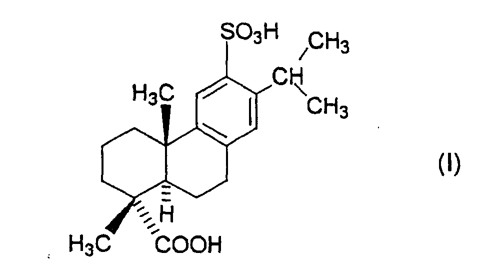 or a pharmaceutically acceptable salt thereof.

2. The agent for prophylaxis or treatment according to claim 1, wherein the inflammatory disease is one in mucosa of oral cavity.

3. The agent for prophylaxis or treatment according to claim 1, wherein the inflammatory disease is aphtha and /or ulcer in mucosa of oral cavity.

4. The agent for prophylaxis or treatment according to claim 1, wherein the inflammatory disease is stomatitis.

5. The agent for prophylaxis or treatment according to claim 1, wherein the inflammatory disease is aphthous stomatitis.

6. The agent for prophylaxis or treatment according to claim 1, wherein the inflammatory disease is pharyngitis.

7. The agent for prophylaxis or treatment according to claim 1, wherein the pharmaceutically acceptable salt of the compound represented by the formula (I) is mono sodium salt.

8. The agent for prophylaxis or treatment according to claim 1 or 2, wherein the preparation is a gargle, a liniment, a patch, a spray, a troche or a buccal.

9. A use of a compound of the formula (I): 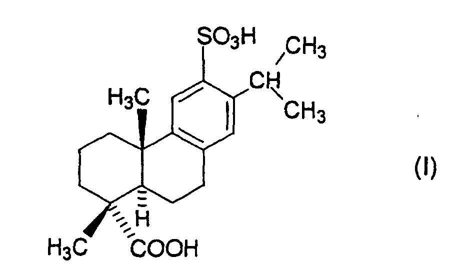 or a pharmaceutically acceptable salt thereof as an active ingredient in the preparation of an agent for prophylaxis or treatment of inflammatory diseases in mucosa of oral cavity, pharynx or larynx.

10. A method for prophylaxis or treatment of inflammatory diseases, which comprises administering an effective amount of a compound of the formula (I) 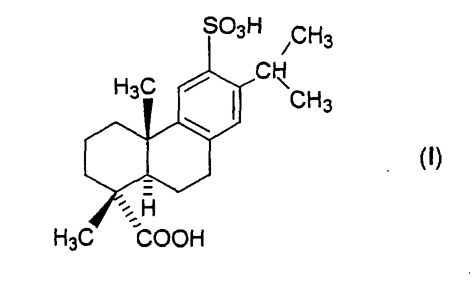 or a pharmaceutically acceptable salt thereof to a patient being suffering from an inflammatory disease in mucosa of oral cavity, pharynx or larynx on said region.
